(19) Europäisches Patentamt
European Patent Office
Office européen des brevets

(11) EP 0 527 235 B1

(12) **EUROPEAN PATENT SPECIFICATION**

(45) Date of publication and mention of the grant of the patent:
**29.10.1997 Bulletin 1997/44**

(21) Application number: **92906157.0**

(22) Date of filing: **28.02.1992**

(51) Int. Cl.$^6$: **C08B 3/16**, C08B 11/193, C08B 13/00, C08B 15/06, B01J 20/22, B01J 20/26, C07B 57/00, G01N 30/48, C08B 37/00

(86) International application number:
**PCT/JP92/00233**

(87) International publication number:
**WO 92/15616 (17.09.1992 Gazette 1992/24)**

(54) **NOVEL POLYSACCHARIDE DRIVATIVE AND SEPARATING AGENT**
NEUE POLYSACCHARIDVERBINDUNG UND TRENNMITTEL
NOUVEAU DERIVE DE POLYSACCHARIDE ET AGENT SEPARATEUR

(84) Designated Contracting States:
**CH DE FR GB IT LI**

(30) Priority: **28.02.1991 JP 34049/91**

(43) Date of publication of application:
**17.02.1993 Bulletin 1993/07**

(73) Proprietor: **DAICEL CHEMICAL INDUSTRIES, LTD.**
**Sakai-shi Osaka-fu 590 (JP)**

(72) Inventor: **OKAMOTO, Yoshio,**
**2-66-222, Yatamachi**
**Higashi-ku**
**Aichi 461 (JP)**

(74) Representative: **Grünecker, Kinkeldey, Stockmair & Schwanhäusser**
**Anwaltssozietät**
**Maximilianstrasse 58**
**80538 München (DE)**

(56) References cited:

| | |
|---|---|
| EP-A- 0 156 382 | EP-A- 0 238 044 |
| EP-A- 0 319 938 | EP-A- 0 384 167 |
| EP-A- 0 436 722 | JP-A- 1 203 402 |
| JP-A-61 157 502 | JP-A-61 233 633 |
| JP-A-62 135 450 | JP-A-63 156 538 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 527 235 B1

## Description

The present invention relates to a polysaccharide derivative which is extremely useful as a material for optical resolution, and a separating agent comprising said polysaccharide derivative.

It has already been known that various racemic modifications can be optically resolved by liquid chromatography using a column packed with a polysaccharide derivative, as disclosed, for example, in Journal of Liquid Chromatography, 9 (2 & 3), 313 to 340 (1986), Japanese Patent Laid-Open No. 142930/1985 and the corresponding patents EP-A 147804 and US-A 4818394, and Japanese Patent Laid-Open No. 178101/1988 and the corresponding patent EP-A 238044. EP-A-436722 (which is prior art according to Art. 54(3) EPC), EP-A-384167, EP-A-319938 and EP-A-156382 disclose polysaccharide derivatives wherein part or the whole of the hydroxyl or amino groups contained therein are displaced by two or more kinds of substituents.

It is the object of the present invention to provide a polysaccharide derivative and a separating agent having an improved resolving power.

Said object is achieved by a polysaccharide derivative in which part or the whole of the hydroxyl or amino groups contained therein are displaced by a substituent having an aromatic group substituted by an electron-donating group and a substituent having an aromatic group substituted by an electron-attractive group, as well as a separating agent comprising said polysaccharide derivative.

The present invention will now be described in detail.

The substituents displacing the hydroxyl groups or the amino groups contained in a polysaccharide according to the present invention are as follows:

(1) substituents for hydroxyl group

combinations of any groups selected from those represented by the formulas:

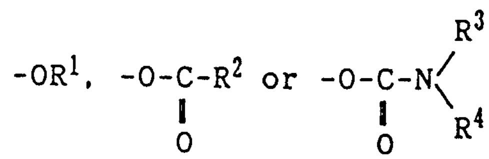

, , or

wherein $R^1$ represents an alkyl group such as a methyl, ethyl, propyl, isopropyl, butyl or t-butyl group, a substituted alkyl group wherein the substituent is a halogen or the like, an aromatic group such as a phenyl group, a substituted aromatic group wherein the substituent is e.g. an alkyl group having 1 to 8 carbon atoms or a halogen, or an aralkyl group such as a benzyl or phenethyl group; $R^2$, $R^3$ and $R^4$ each represent a hydrogen atom, an alkyl group such as a methyl, ethyl, propyl, isopropyl, butyl or t-butyl group, a substituted alkyl group wherein the substituent is e.g. a halogen, an aromatic group such as a phenyl group, a substituted aromatic group wherein the substituent is e.g. an alkyl group having 1 to 8 carbon atoms, or a halogen atom, or an aralkyl group such as a benzyl or phenethyl group.

(2) substituents for amino group

combinations of any groups selected from those represented by the formula:

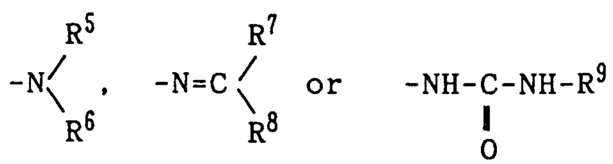

, , or

wherein $R^5$ and $R^6$ each represent a hydrogen atom, an alkyl group such as a methyl, ethyl, propyl, isopropyl, butyl or t-butyl group, a substituted alkyl group wherein the substituent is a halogen atom or the like, an aromatic group such as a phenyl group, a substituted aromatic group wherein the substituent is e.g. an alkyl group having 1 to 8 carbon atoms or a halogen atom, an aralkyl group such as a benzyl or phenethyl group, or an acyl group such as an acetyl or phenacetyl group, with the proviso that a case wherein $R^5$ and $R^6$ are simultaneously hydrogen atoms is excepted; and $R^7$, $R^8$ and $R^9$ each represent a hydrogen atom, an alkyl group such as a methyl, ethyl, propyl, isopropyl, butyl or t-butyl group, a substituted alkyl group wherein the substituent is e.g. a halogen, an aromatic group such as a phenyl group, a substituted aromatic group wherein the substituent is e.g. an alkyl group having 1 to 8 carbon atoms or a halogen atom, or an aralkyl group such as a benzyl or phenethyl group.

Substituents having an aromatic group substituted by an electron-donating group at the 2- and/or 3-position are preferable. The electron-donating group includes alkyl groups. The combination of a 3,5-dimethylphenylcarbamate group represented by the following formula (I) with a 3,5-dichlorophenylcarbamate group represented by the following formula (II) is preferable:

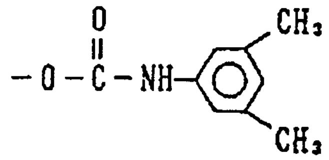

··· (I)

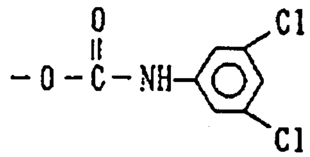

···.(II)

It is preferable that the weight ratio of the 3,5-dimethylphenylcarbamate group (I) to the 3,5-dichlorophenylcarbamate group (II) ranges from 9 : 1 to 1 : 9.

The polysaccharide derivative according to the present invention has at least two different substituents. Particularly, a polysaccharide derivative in which the substituents present at the 2- and/or 3-position are different from that present at the 6-position is preferable. The degree of displacement of the polysaccharide derivative according to the present invention is at least 40%, preferably at least 50%, and still preferably at least 85%.

Although the polysaccharide according to the present invention may be any of synthetic, natural and modified natural ones so far as it is optically active, it is preferably one having a highly ordered bonding mode. Particular examples thereof include α-1,4-glucans (such as amylose and amylopectin), α-1,6-glucans (such as dextran), β-1,4-glucans (such as cellulose), β-1,6-glucans (such as pustulan), β-1,3-glucans (such as curdlan and schizophyllan), β-1,3-glucans, β-1,2-glucans (such as crown gall polysaccharide), β-1,4-galactans, β-1,4-mannans, α-1,6-mannans, β-1,2-fructans (such as inulin), β-2,6-fructans (such as levan), β-1,4-xylans, β-1,3-xylans, β-1,4-chitosan, β-1,4-N-acetylchitosan (i.e., chitin), pullulan, agarose, alginic acid and amylose-containing starch, among which amylose, cellulose, β-1,4-chitosan, chitin, β-1,4-mannans, β-1,4-xylans, inulin and curdlan are particularly preferable because high-purity polysaccharides can be easily obtained therefrom.

The number-average degree of polymerization of the polysaccharide (the average number of pyranose or furanose rings contained in one polysaccharide molecule) is 5 or above, preferably 10 or above and the upper limit thereof is 2000, preferably 500 from the standpoint of handleability.

The displacement of the hydroxyl groups or the amino groups of a polysaccharide by the substituents described above can be conducted by the following processes:

(1) Process for displacement of hydroxyl groups

A) displacement to ester

The hydroxyl groups of the polysaccharide according to the present invention can be easily displaced to ester groups by the process in which the corresponding carboxylic acids ($R^2COOH$) are reacted with thionyl chloride or oxalyl chloride to form acid chlorides and then the acid chlorides are reacted with the corresponding polysaccharide in pyridine.

B) displacement to carbamate

The hydroxyl groups of the polysaccharide according to the present invention can be easily displaced to carbamate groups by any conventional process for preparing a urethane from an alcohol and an isocyanate. For example, they can be displaced to carbamate groups by reacting the corresponding isocyanates ($R^3NCO$) with a polysaccharide in a suitable solvent in the presence of a catalyst, e.g., a Lewis base such as a tertiary amine or a Lewis acid such as a tin compound. The isocyanate can be easily prepared by, for example, reacting the amino group of the corresponding aniline derivative with phosgene.

C) displacement to ether

The hydroxyl groups of the polysaccharide according to the present invention can be easily displaced to ether groups by reacting the corresponding halides ($R^1X$ wherein X represents a halogen atom) with the corresponding polysaccharide in a solvent such as dioxane or pyridine in the presence of a base such as potassium hydroxide or potassium t-butoxide.

(2) Process for displacement of amino groups

(D) displacement to vinylidene

The amino groups of the polysaccharide according to the present invention can be displaced to vinylidene groups by any conventional process for preparing a Schiff base from an amine and a carbonyl compound.

(E) displacement to amide

The amino groups of the polysaccharide according to the present invention can be displaced to amide groups by any conventional process for preparing an amide from an amine and an acid chloride.

(F) displacement to urea

The amino groups of the polysaccharide according to the present invention can be displaced to urea by any conventional process for preparing urea from an amine and an isocyanate.

According to the present invention, the hydroxyl groups or the amino groups of a polysaccharide must be displaced by at least two kinds of substituents, which can be attained by simultaneously reacting two or more reactants as described above with a polysaccharide or by stepwise reacting them with a polysaccharide to thereby displace specified hydroxyl group(s) or amino group(s) by specified substituents.

Among the polysaccharide derivatives of the present invention, a polysaccharide derivative having a 3,5-dimethylphenylcarbamate group represented by the above formula (I) and a 3,5-dichlorophenylcarbamate group represented by the above formula (II) can be prepared by reacting a polysaccharide with 3,5-dimethylphenyl isocyanate and 3,5-dichlorophenyl isocyanate. This reaction can be conducted by any conventional process for preparing a urethane from an alcohol and an isocyanate. For example, the above polysaccharide derivative can be prepared by reacting the corresponding isocyanates with a polysaccharide in a suitable solvent in the presence of a Lewis base such as a tertiary amine or a Lewis acid such as a tin compound as a catalyst.

The polysaccharide derivative of the present invention is an extremely useful substance as a functional material, particularly as a packing for optical resolution, i.e., a separating agent.

The separation of a mixture of compounds or a mixture of optical isomers by the use of the polysaccharide derivative according to the present invention as a separating agent is generally conducted by a chromatographic process such as gas chromatography, liquid chromatography or thin-layer chromatography. Alternatively, it can also be conducted according to a membrane separation process by the use of a membrane containing the polysaccharide derivative according to the present invention.

When the polysaccharide derivative of the present invention is applied to liquid chromatography as a separating agent, it is favorable from the standpoints of easiness and simplicity that the polysaccharide derivative be packed into a column in powdery form. It is preferable that the polysaccharide derivative of the present invention be pulverized or converted into beads and it is still preferable that the resulting particle be porous. Further, it is desirable that the polysaccharide derivative be supported on a carrier for the purpose of improving the pressure resistance of the separating agent, preventing the separating agent from undergoing swelling or shrinkage due to the replacement of the solvent, and improving the number of theoretical plates.

When the polysaccharide derivative of the present invention is used in powdery form, the sizes of the particle and the carrier are 1 μm to 1 mm, preferably 1 μm to 300 μm, though they vary depending upon the size of the column to be used. It is desirable that the carrier be porous with the mean pore diameter being 1 nm (10 Å) to 100 μm, preferably 5 to 5000 nm (50 Å to 50000 Å). The amount of the polysaccharide derivative to be supported on a carrier is 1 to 100% by weight, preferably 5 to 50% by weight based on the carrier.

The polysaccharide derivative may be supported on a carrier by a chemical or physical process. The physical process includes a process of dissolving the polysaccharide derivative in a solvent wherein it is soluble, mixing the obtained solution with a carrier and blowing out the solvent with an air stream either under a reduced pressure or under heating and a process of dissolving the polysaccharide derivative in a solvent wherein it is soluble, mixing the obtained solution with a carrier and treating the obtained mixture with another solvent in which the polysaccharide derivative is insoluble to diffuse the former solvent in the latter solvent. The separating agent thus prepared may be further subjected to suitable treatment such as heating, addition of a solvent or washing in order to improve the separating power thereof.

The carrier to be used in the present invention may be a porous organic carrier or a porous inorganic carrier, with the use of a porous inorganic carrier being preferable. Suitable examples of the porous organic carrier include polymers such as polystyrene, polyacrylamide and polyacrylate. On the other hand, suitable examples of the porous inorganic carrier include silica, alumina, magnesia, glass, kaolin, titanium oxide and silicates, which may be surface-treated for the purpose of improving the affinity thereof with the polysaccharide derivative and the surface characteristics of the carrier itself. The surface treatment includes a treatment with an organic silane compound and a treatment with plasma polymerization.

When the polysaccharide derivative of the present invention is used in liquid chromatography or thin-layer chromatography, the developer to be used is not particularly limited except for ones which dissolve the polysaccharide derivative or react with it. When the polysaccharide derivative is chemically supported on a carrier or insolubilized by crosslinking, all developers are usable except ones reactive with it.

When the polysaccharide derivative is used in thin layer chromatography, a layer comprising the separating agent in the form of a particle having a size of 0.1 μm to 0.1 mm and, if necessary, a small amount of a binder is formed on a base in a thickness of 0.1 μm to 100 mm.

Further, when the polysaccharide derivative is applied to a membrane separation process, it is used as a hollow yarn or a film.

The present invention will now be described in more detail by referring to the following examples.

## Example 1

0.722 g of cellulose (Avicel, a product of Merck), 0.988 g (6.71 mmol, 50% by equivalent) of 3,5-dimethylphenyl isocyanate and 1.243 g (6.68 mmol, 50% by equivalent) of 3,5-dichlorophenyl isocyanate were added to 35 ml of pyridine (which had been simple-distilled and dried with KOH). The obtained mixture was maintained at about 100°C for 15 hours to conduct a reaction, followed by the addition of 0.485 g (3.30 mmol) of 3,5-dimethylphenyl isocyanate and 0.613 g (3.30 mmol) of 3,5-dichlorophenyl isocyanate. The obtained mixture was stirred at 110°C for 10 hours and poured into methanol to give precipitates, which were collected on a glass filter, washed with methanol, and vacuum-dried at 60°C for 2 hours. Thus 2.761 g of a cellulose derivative was obtained in a yield of 93.2%. Hereinafter, this cellulose derivative is referred to as "Cellulose derivative (1)".

## Example 2

1.01 g of cellulose (Avicel, a product of Merck), 1.83 g (12.45 mmol, 67% by equivalent) of 3,5-dimethylphenyl isocyanate and 1.16 g (6.24 mmol, 33% by equivalent) of 3,5-dichlorophenyl isocyanate were added to 35 ml of pyridine (which had been simple-distilled and dried over with KOH). The obtained mixture was maintained at about 100°C for 15 hours to conduct a reaction, followed by the addition of 15 ml of pyridine, 0.54 g (3.73 mmol) of 3,5-dimethylphenyl isocyanate and 0.34 g (1.83 mmol) of 3,5-dichlorophenyl isocyanate. The obtained mixture was stirred at 100°C for 24 hours and poured into methanol to give precipitates, which were collected on a glass filter, washed with methanol, and vacuum-dried at 60°C for 2 hours. Thus 3.520 g of a cellulose derivative was obtained in a yield of 100%. Hereinafter, this cellulose derivative is referred to as "Cellulose derivative (2)".

## Example 3

1.68 g of tritylcellolose (degree of substitution by trityl group: 43%) was dissolved in 20 ml of pyridine, followed by the addition of 1.75 g (excess based on the residual hydroxyl group) of 3,5-dimethylphenyl isocyanate. The obtained mixture was maintained at about 100°C for 9 hours to conduct a reaction. The reaction mixture was poured into methanol to give precipitates, which were collected on a glass filter and washed with methanol.

1.45 g of the cellulose derivative thus prepared was suspended in 40 ml of methanol, followed by the addition of four drops of concentrated hydrochloric acid. The obtained mixture was stirred at room temperature for 20 hours to remove the trityl groups from the cellulose derivative. The resulting cellulose derivative was collected on a glass filter, washed with methanol and vacuum-dried at 60°C for 2 hours (weight of product: 0.770 g, yield: 92.3%).

0.77 g of the cellulose derivative prepared above and 1.10 g (5.93 mmol, excess) of 3,5-dichlorophenyl isocyanate were dissolved in 15 ml of pyridine. The obtained solution was maintained at about 100°C for 15 hours to conduct a reaction. The reaction mixture was poured into methanol to give precipitates, which were collected by centrifuging, washed with methanol repeatedly, dried in a desiccator for about 3 hours, and vacuum-dried at 60°C for 2 hours. Thus 1.045 g of cellulose derivative was obtained in a yield of 86.3%.

The obtained cellulose derivative was selectively modified with a 3,5-dichlorophenylcarbamate group at the 6-position and 3,5-dimethylphenylcarbamate groups at the 2- and 3-positions, which is hereinafter referred to as "Cellulose derivative (3)".

The results of elemental analysis of the Cellulose derivatives (1) to (3) prepared in Examples 1 to 3 are given in Table 1. For reference, the results of elemental analysis of cellulose tris(3,5-dichlorophenylcarbamate) (reference 1) and cellulose tris(3,5-dimethylphenylcarbamate) (reference 2) are also given in Table 1.

Table 1

| Cellulose derivative | Results of elemental analysis | | | | Content of Cl* (%) |
|---|---|---|---|---|---|
| | C% | H% | N% | Cl% | |
| (1) | 51.01 | 3.79 | 6.10 | 19.46 | 62.19 |
| (2) | 56.09 | 4.72 | 5.89 | 12.42 | 42.39 |
| (3) | 52.71 | 3.81 | 5.88 | 18.73 | 59.57 |
| ref. 1 | 44.66 | 2.64 | 5.79 | 29.30 | - |
| ref. 2 | 65.66 | 6.18 | 6.96 | - | - |

note)
*: the content of 3,5-dichlorophenylcarbamate group calculated from the Cl content determined by elemental analysis.

Example 4

0.75 g of each of the Cellulose derivatives (1) to (3) prepared in Examples 1 to 3 and the referential ones 1 and 2 was dissolved in 15 ml of tetrahydrofuran. The obtained solutions were each supported on 3.0 g of silica gel (a product of Merck, SI4000) treated with 3-aminopropyltriethoxysilane to give various packings.

These packings were each packed into a column having a length of 25 cm and an inner diameter of 0.46 cm with the use of a hexane/liquid paraffin (2 : 1) mixture.

Various racemic compounds listed in Tables 2 and 3 were resolved by the use of the columns prepared above. The results are given in Tables 2 and 3.

The capacity ratio (k') and separation factor (α) given in the Tables were calculated by the following formulas, respectively:

$$\text{capacity ratio } (k') = \frac{\text{retention time of separated compound - dead time}}{\text{dead time}}$$

$$\text{separation factor } (\alpha) = \frac{\text{capacity ratio of more strongly adsorbed compound}}{\text{capacity ratio of more weakly adsorbed compound}}$$

Table 2

| Racemic modification | Ref. 1 | | Ref. 2 | | Cellulose deriv. (1) | | Cellulose deriv. (2) | | Cellulose deriv. (3) | |
|---|---|---|---|---|---|---|---|---|---|---|
| | k' | α | k' | α | k' | α | k' | α | k' | α |
| 1. 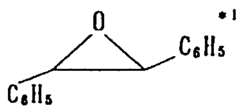 *1 | 0.56(+) | 1.84 | 0.74(-) | 1.68 | 0.36(+) | 2.03 | 0.37(-) | ~1 | 0.35(+) | 1.80 |
| 2. 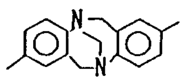 *1 | 0.87(+) | 1.65 | 0.97(+) | 1.32 | 0.53(+) | 1.84 | 0.46(+) | 1.32 | 0.52(+) | 1.39 |
| 3. $Co(acac)_3$ *1 | 0.76(+) | 1.82 | 0.42(+) | ~1 | 0.36(+) | 1.69 | 0.28(+) | 1.41 | 0.47(+) | 1.50 |
| 4. 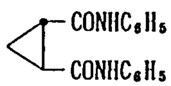 *1 | 0.59(+) | 1.41 | 0.83(+) | 3.17 | 0.66(+) | ~1 | 0.49(+) | 1.17 | 0.68(-) | 1.20 |
| 5. 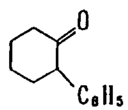 *1 | 2.65(-) | 1.26 | 1.17(-) | 1.15 | 1.47(-) | 1.26 | 0.69(-) | 1.22 | 0.97(-) | 1.28 |
| 6. 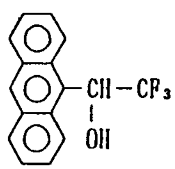 *1 | 0.28(-) | 1.38 | 2.13(-) | 2.59 | 0.37(-) | 1.50 | 0.45(-) | 1.72 | 0.44(-) | 1.37 |

Table 3

| Racemic modification | Ref. 1 | | Ref. 2 | | Cellulose deriv. (1) | | Cellulose deriv. (2) | | Cellulose deriv. (3) | |
|---|---|---|---|---|---|---|---|---|---|---|
| | k' | α | k' | α | k' | α | k' | α | k' | α |
| 7. 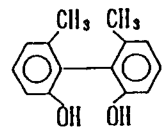 *1 | 1.62(+) | 1.11 | 2.36(−) | 1.83 | 1.48(−) | 1.36 | 1.01(−) | 1.82 | 1.08(−) | 1.30 |
| 8. 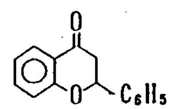 *1 | 1.55(−) | 1.20 | 1.47(−) | 1.41 | 1.03(−) | 1.20 | 0.63(−) | 1.22 | 0.77(−) | 1.15 |
| 9. 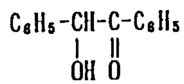 *1 | 3.08(−) | 1.21 | 2.43(+) | 1.58 | 2.08(−) | 1.07 | 1.37(+) | 1.19 | | |
| 10. 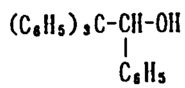 *1 | 0.40(+) | 1.29 | 1.37(+) | 1.34 | 0.47(+) | ~1 | 0.48(+) | ~1 | 0.43(+) | 1.14 |
| 11. 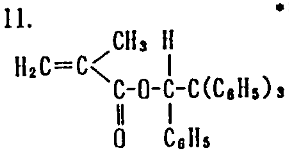 *2 | 0.48(+) | 4.23 | 0.37(−) | 1.73 | 0.52(+) | 2.77 | 0.72(+) | 1.25 | 0.54(+) | 1.68 |

note)

*1 eluent: n-hexane / 2-propanol (90 : 10)

*2 eluent: n-hexane / 2-propanol (98 : 2)

## Claims

1. A polysaccharide derivative in which part or the whole of the hydroxyl or amino groups contained therein are displaced by a substituent having an aromatic group substituted by an electron-donating group and a substituent having an aromatic group substituted by an electron-attractive group.

2. The polysaccharide derivative of claim 1, which has a 3,5-dimethylphenylcarbamate group represented by the following formula (I) and a 3,5-dichlorophenylcarbamate group represented by the following formula (II) as substituents:

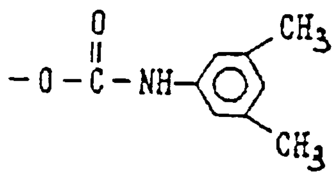

(I)

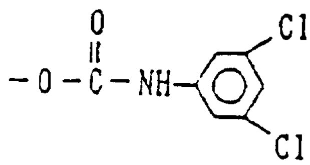

(II)

3. A separating agent which comprises the polysaccharide derivative of claim 1 or 2.

4. A process for resolving a racemic mixture into optical isomers by the use of the separating agent of claim 3.

## Patentansprüche

1. Polysaccharidderivat, wobei, teilweise oder ganz, die enthaltenen Hydroxyl- oder Aminogruppen durch einen Substituenten, enthaltend eine aromatische Gruppe mit einer elektronenspendenden Gruppe, und einen Substituenten, enthaltend eine aromatische Gruppe mit einer elektronenziehenden Gruppe, ersetzt sind.

2. Polysaccharidderivat, nach Anspruch 1, **dadurch gekennzeichnet**, daß als Substituenten eine 3,5-Dimethylphenylcarbamatgruppe, repräsentiert durch die folgende Formel (I) und eine 3,5-Dichlorophenylcarbamatgruppe, repräsentiert durch die folgende Formel (II) vorliegen.

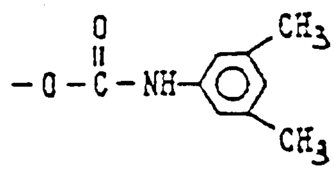

(I)

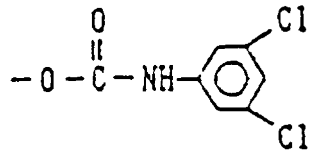

(II)

3. Ein Trennmittel, enthaltend das Polysaccharidderivat nach einem der Ansprüche 1 oder 2.

4. Ein Verfahren zur Trennung einer racemischen Mischung in die optischen Isomere unter Einsatz des Trennmittels nach Anspruch 3.

**Revendications**

1. Dérivé de polysaccharide dans lequel une partie ou la totalité des groupes hydroxyle ou amino qu'il contient sont remplacés par un substituant ayant un groupe aromatique substitué par un groupe donneur d'électrons et un substituant ayant un groupe aromatique substitué par un groupe attracteur d'électrons.

2. Dérivé de polysaccharide selon la revendication 1, qui a, comme substituants, un groupe 3,5-diméthylphénylcarbamate représenté par la formule (I) suivante et un groupe 3,5-dichlorophénylcarbamate représenté par la formule (II) su

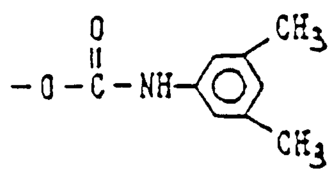

(I)

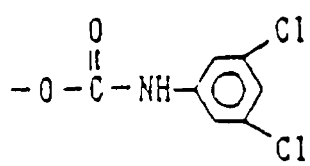

(II)

ivante :

3. Agent de séparation qui comprend le dérivé de polysaccharide de la revendication 1 ou 2.

4. Procédé pour dédoubler un mélange racémique en isomères optiques par utilisation de l'agent de séparation de la revendication 3.